(19)

Europäisches Patentamt
European Patent Office
Office européen des brevets

(11) **EP 4 394 442 A1**

(12) **EUROPEAN PATENT APPLICATION**

(43) Date of publication:
**03.07.2024 Bulletin 2024/27**

(21) Application number: **23220440.4**

(22) Date of filing: **28.12.2023**

(51) International Patent Classification (IPC):
**G01S 7/52** (2006.01)   **G01S 15/89** (2006.01)
**A61B 8/00** (2006.01)

(52) Cooperative Patent Classification (CPC):
**G01S 7/5202; A61B 8/54; G01S 15/8915;**
**G01S 15/8927;** A61B 8/12; A61B 8/4494;
G01S 15/8918; G01S 15/892

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **29.12.2022   CN 202211715857**
**12.12.2023   CN 202311707871**

(71) Applicant: **Wuhan United Imaging Healthcare Co.,**
**Ltd.**
**WuHan, Hubei 430206 (CN)**

(72) Inventors:
• LI, Tong
  **Wuhan, 430206 (CN)**
• HE, Cheng
  **Wuhan, 430206 (CN)**

(74) Representative: **Dai, Simin**
**Reyda IP**
**157, Quai du Président Roosevelt**
**Appt A073**
**92130 Issy-les-Moulineaux (FR)**

(54) **CONTROL METHOD FOR MEDICAL PROBE, IMAGING METHOD, AND ULTRASOUND SYSTEM**

(57)    The present disclosure discloses a control method for medical probe, an imaging method, and an ultrasound system. The control method includes: determining a delay correspondence matching a deflection angle; triggering the transducer to emit a scanning line beam with a deflection angle according to the delay correspondence, so that scanning line beams emitted by array elements included in the transducer are focused on one focus in one emission, where scanning line beams with the same deflection angle are emitted based on the same delay correspondence. In the present disclosure, the transducer is triggered to emit a scanning line beam with a deflection angle by sharing a delay correspondence, which can reduce a number of delay correspondences stored and save storage space, and there is no need to re-calculate an emission delay time of each array element based on the focus positions, which can improve the emission efficiency.

FIG. 1

Determine a delay correspondence matching a deflection angle — 101

Trigger a transducer to emit a scanning line beam with a deflection angle according to the delay correspondence, so that array elements included in the transducer are focused on one focus in one emission — 102

EP 4 394 442 A1

**Description**

The present application claims a priority of Chinese patent application No. 202211715857.2, the application date of which is December 29, 2022, and a priority of Chinese patent application No. 202311707871.2, the application date of which is December 12, 2023. The contents of these Chinese patent applications are incorporated herein by reference in their entireties.

**TECHNICAL FIELD**

The present disclosure relates to the technical field of medical imaging, and in particular to, a control method for a medical probe, an imaging method, and an ultrasound system.

**BACKGROUND**

Ultrasound systems are now commonly used in many clinical applications such as obstetrics, gynecology, and urology. In order to improve the flaw detection sensitivity and resolution of an ultrasound probe, the ultrasound probe uses single-focus focusing imaging. When the single-focus focusing imaging is used, each time a focus position is changed, each array element needs to emit a scanning line beam at a new deflection angle, and an emission delay time of each emitting array element needs to be recalculated, which is relatively slow and requires a large amount of calculation. In order to reduce an amount of calculation, the prior art also uses a preset emission delay curve to determine the emission delay time of the array element. Each emission corresponds to one delay correspondence. If there are M deflection angles, N emissions may be performed at M deflection angles. M*N emission delay curves need to be stored, which will increase the storage space of an ultrasound system.

**CONTENT OF THE PRESENT INVENTION**

The technical problem to be solved by the present disclosure is to provide a control method for a medical probe, an imaging method, and an ultrasound system, to overcome the above-mentioned defects in the prior art.
The present disclosure solves the above-mentioned technical problem through the following technical solutions.
According to a first aspect, a control method for a medical probe is provided, the method is applied to an ultrasound system, the ultrasound system includes a transducer, and the control method includes:

> determining a delay correspondence matching a deflection angle; and
> triggering the transducer to emit a scanning line beam with a deflection angle according to the delay correspondence, so that scanning line beams emitted by array elements included in the transducer are focused on one focus in one emission, where scanning line beams with the same deflection angle are emitted based on the same delay correspondence, and the delay correspondence represents a correspondence between an array element number and an emission delay time.

Optionally, the transducer includes a transducer array, the transducer array includes a scanning unit, and the triggering the transducer to emit a scanning line beam with a deflection angle according to the delay correspondence includes:

> determining, based on a boundary position of an effective emission aperture of one emission by the scanning unit and the delay correspondence, an emission delay time of an array element included in the scanning unit;
> triggering the array element to emit the scanning line beam with the emission delay time.

Optionally, the determining, based on a boundary position of an effective emission aperture of one emission by the scanning unit and the delay correspondence, an emission delay time of an array element included in the scanning unit includes:

> determining, based on the boundary position of the effective emission aperture, an index between each array element included in the scanning unit and each array element number represented by the delay correspondence;
> matching the emission delay time of the array element from the delay correspondence based on the index.

Optionally, the boundary position includes a starting array element position, and the determining, based on the boundary position of the effective emission aperture, an index between each array element included in the scanning unit and each array element number represented by the delay correspondence includes:

determining the starting array element position and a first-array element position of the scanning unit;
determining, based on the starting array element position, the first-array element position, and a starting array element number for an effective delay in the delay correspondence, an index between each array element included in the scanning unit and each array element number represented by the delay correspondence.

Optionally, the transducer includes at least two transducer arrays, and the triggering the transducer to emit a scanning line beam with a deflection angle according to the delay correspondence includes:
triggering each transducer array to emit a scanning line beam with a deflection angle according to the delay correspondence, so that imaging areas of two adjacently arranged transducer arrays having a same imaging section type have overlapping areas, where the imaging areas include at least one focus.

Optionally, the transducer includes a linear transducer array, and the delay correspondence is pre-established in the following manner of: determining a focus position based on a focus depth of the linear transducer array and the deflection angle, determining an emission delay time of each array element based on the focus position and a position of an array element included in the linear transducer array, and establishing the delay correspondence; and/or the transducer includes a convex transducer array, and the delay correspondence is pre-established in the following manner of: determining a focus position based on a radius and a focus depth of the convex transducer array and the deflection angle, determining an emission delay time of each array element based on the focus position and a position of an array element included in the convex transducer array, and establishing the delay correspondence.

Optionally, the ultrasound system further includes emission channels, the transducer includes a transducer array, and the triggering the transducer to emit a scanning line beam with a deflection angle according to the delay correspondence includes:
mapping the emission channels to transducer arrays in sequence, and triggering the mapped transducer arrays through the emission channels to emit a scanning line beam with a deflection angle according to the delay correspondence.

According to a second aspect, an imaging method is provided, the method is applied to an ultrasound system, the ultrasound system includes a transducer, and the imaging method includes:

determining a delay correspondence matching a deflection angle;
triggering the transducer to emit a scanning line beam with a deflection angle according to the delay correspondence, so that scanning line beams emitted by array elements included in the transducer are focused on one focus in one emission, where scanning line beams with the same deflection angle are emitted based on the same delay correspondence, and the delay correspondence represents a correspondence between an array element number and an emission delay time;
obtaining scanned image data collected by the transducer;
performing image reconstruction on the scanned image data to obtain a medical image.

Optionally, the transducer includes a transducer array, the transducer array includes a scanning unit, and the triggering the transducer to emit a scanning line beam with a deflection angle according to the delay correspondence includes:

determining, based on a boundary position of an effective emission aperture of one emission by the scanning unit and the delay correspondence, an emission delay time of an array element included in the scanning unit;
triggering the array element to emit the scanning line beam with the emission delay time.

Optionally, the determining, based on a boundary position of an effective emission aperture of one emission by the scanning unit and the delay correspondence, an emission delay time of an array element included in the scanning unit includes:

determining, based on the boundary position of the effective emission aperture, an index between each array element included in the scanning unit and each array element number represented by the delay correspondence;
matching the emission delay time of the array element from the delay correspondence based on the index.

Optionally, the boundary position includes a starting array element position, and the determining, based on the boundary position of the effective emission aperture, an index between each array element included in the scanning unit and each array element number represented by the delay correspondence includes:

determining the starting array element position and a first-array element position of the scanning unit;
determining, based on the starting array element position, the first-array element position, and a starting array element number for an effective delay in the delay correspondence, an index between each array element included in the scanning unit and each array element number represented by the delay correspondence.

Optionally, the transducer includes at least two transducer arrays, and the triggering the transducer to emit a scanning line beam with a deflection angle according to the delay correspondence includes:

triggering each transducer array to emit a scanning line beam with a deflection angle according to the delay correspondence, so that imaging areas of two adjacently arranged transducer arrays having a same imaging section type have overlapping areas, where the imaging areas include at least one focus.

Optionally, the transducer includes a linear transducer array, and a focus position of emission by the linear transducer array is determined based on a focus depth and the deflection angle; the delay correspondence is pre-established in the following manner of: determining the focus position based on the focus depth of the linear transducer array and the deflection angle, determining an emission delay time of each array element based on the focus position and a position of an array element included in the linear transducer array, and establishing the delay correspondence; and/or the transducer includes a convex transducer array, and the delay correspondence is pre-established in the following manner of: determining a focus position based on a radius and a focus depth of the convex transducer array and the deflection angle, determining an emission delay time of each array element based on the focus position and a position of an array element included in the convex transducer array, and establishing the delay correspondence.

Optionally, the performing image reconstruction on the scanned image data includes:

performing image reconstruction on scanned image data having a same imaging section type, to obtain a medical image corresponding to the imaging section type.

Optionally, the ultrasound system further includes emission channels, the transducer includes a transducer array, and the triggering the transducer to emit a scanning line beam with a deflection angle according to the delay correspondence includes:

mapping the emission channels to transducer arrays in sequence, and triggering the mapped transducer arrays through the emission channels to emit a scanning line beam with a deflection angle according to the delay correspondence.

According to a third aspect, an ultrasound system is provided, and the ultrasound system includes a transducer and a controller.

The controller is configured to determine a delay correspondence matching a deflection angle, and trigger the transducer to emit a scanning line beam with a deflection angle according to the delay correspondence, so that scanning line beams emitted by array elements included in the transducer are focused on one focus in one emission, where scanning line beams with the same deflection angle are emitted based on the same delay correspondence, and the delay correspondence represents a correspondence between an array element number and an emission delay time.

Optionally, the transducer includes a transducer array, the transducer array includes a scanning unit, and the controller is configured to determine, based on a boundary position of an effective emission aperture of one emission by the scanning unit and the delay correspondence, an emission delay time of an array element included in the scanning unit, and trigger the array element to emit the scanning line beam with the emission delay time.

Optionally, the controller is configured to determine, based on the boundary position of the effective emission aperture, an index between each array element included in the scanning unit and each array element number represented by the delay correspondence, and match the emission delay time of the array element from the delay correspondence based on the index.

Optionally, the transducer includes at least two transducer arrays, and the controller is configured to trigger each transducer array to emit a scanning line beam with a deflection angle according to the delay correspondence, so that imaging areas of two adjacently arranged transducer arrays having a same imaging section type have overlapping areas, where the imaging areas include at least one focus.

Optionally, the transducer includes a first transducer array and a second transducer array of different types; there are at least two first transducer arrays, and the first transducer arrays are arranged on both sides of a length direction of the second transducer array.

Optionally, the transducer includes a linear transducer array, and the delay correspondence is pre-established in the following manner of: determining a focus position based on a focus depth of the linear transducer array and the deflection angle, determining an emission delay time of each array element based on the focus position and a position of an array element included in the linear transducer array, and establishing the delay correspondence; and/or the transducer includes a convex transducer array, and the delay correspondence is pre-established in the following manner of: determining a focus position based on a radius and a focus depth of the convex transducer array and the deflection angle, determining an emission delay time of each array element based on the focus position and a position of an array element included in the convex transducer array, and establishing the delay correspondence.

Optionally, the ultrasound system further includes emission channels, the transducer includes a transducer array, and the controller is configured to map the emission channels transducer arrays in sequence, and trigger the mapped transducer arrays through the emission channels to emit a scanning line beam with a deflection angle according to the delay correspondence.

Optionally, the ultrasound system further includes a switch array, the transducer is connected to the emission channels through the switch array, and the controller switches states of switches in the switch array to map the emission channels to the transducer array in sequence.

According to a fourth aspect, an electronic device is provided, including a memory, a processor, and a computer program stored in the memory and capable of running on the processor, where when the processor executes the computer program, the method described in the first aspect or the second aspect is implemented.

According to a fifth aspect, a computer-readable storage medium with a computer program stored therein is provided, where when the computer program is executed by a processor, the method described in the first aspect or the second aspect is implemented.

On the basis of conforming to common knowledge in the art, the above-mentioned preferred conditions can be arbitrarily combined to obtain various preferred embodiments of the present disclosure.

The positive effects of the present disclosure lie in: In the present disclosure, the transducer is triggered to emit a scanning line beam with a deflection angle by sharing a delay correspondence, which can reduce a number of delay correspondences stored and save storage space, and for different focus positions, there is no need to re-calculate an emission delay time of each array element based on the focus positions, which can improve the emission efficiency.

## BRIEF DESCRIPTION OF THE DRAWINGS

FIG. 1 is a flowchart of a control method for a medical probe according to an exemplary embodiment of the present disclosure;

FIG. 2 is a schematic diagram of a delay sharing emission process of a medical probe according to an exemplary embodiment of the present disclosure;

FIG. 3 is a schematic diagram of a delay sharing emission process of another medical probe according to an exemplary embodiment of the present disclosure;

FIG. 4a is a schematic diagram of a structure of a medical probe according to an exemplary embodiment of the present disclosure;

FIG. 4b is a schematic diagram of a structure of another medical probe according to an exemplary embodiment of the present disclosure;

FIG. 4c is a schematic diagram of a structure of another medical probe according to an exemplary embodiment of the present disclosure;

FIG. 5 is a schematic diagram of focusing of a linear transducer array included in a medical probe according to an exemplary embodiment of the present disclosure;

FIG. 6 is a schematic diagram of emission with a deflection angle by a linear transducer array included in a medical probe according to an exemplary embodiment of the present disclosure;

FIG. 7 is a schematic diagram of focusing of a convex transducer array included in a medical probe according to an exemplary embodiment of the present disclosure;

FIG. 8 is a schematic diagram of emission with a deflection angle by a convex transducer array included in a medical probe according to an exemplary embodiment of the present disclosure;

FIG. 9 is a flowchart of another control method for a medical probe according to an exemplary embodiment of the present disclosure;

FIG. 10 is a schematic diagram of an emission timing sequence of a transducer array of a medical probe according to an exemplary embodiment of the present disclosure;

FIG. 11 is a flowchart of an imaging method according to an exemplary embodiment of the present disclosure;

FIG. 12 is a schematic diagram of scan transformation of a linear transducer array included in a medical probe according to an exemplary embodiment of the present disclosure;

FIG. 13 is a schematic diagram of scan transformation of a convex transducer array included in a medical probe according to an exemplary embodiment of the present disclosure;

FIG. 14 is a schematic diagram of modules of an ultrasound system according to an exemplary embodiment of the present disclosure;

FIG. 15 is a schematic diagram of modules of another ultrasound system according to an exemplary embodiment of the present disclosure;

FIG. 16 is a schematic diagram of a structure of an electronic device according to an exemplary embodiment of the present disclosure.

**DETAILED DESCRIPTION OF THE PREFERRED EMBODIMENT**

The present disclosure is further described below by way of examples; however, the present disclosure is not limited to the scope of the described examples.

In the embodiments of the present disclosure, prefixes such as "first" and "second" are used only to distinguish different described objects, and do not limit the position, order, priority, number, or content of the described objects. In the embodiments of the present disclosure, the use of ordinal words and other prefixes used to distinguish the described objects does not limit the described objects. For the statement of the described objects, refer to the claims or the context description in the embodiments. The use of such prefixes should not constitute redundant restrictions. In addition, in the description of the embodiments, the meaning of "a plurality of" is two or more, unless otherwise stated.

An embodiment of the present disclosure provides a control method for a medical probe, the method is applied to an ultrasound system, and the ultrasound system includes a transducer. In this embodiment, the medical probe is controlled to use single-focus focusing imaging. The single-focus focusing imaging means that a scanning line beam emitted by the transducer each time is focused on one focus. Each time a focus position is changed, a scanning line beam needs to be emitted at a new deflection angle, that is, a deflection angle needs to be re-determined if a focus position is switched. The deflection angle is preset according to actual needs.

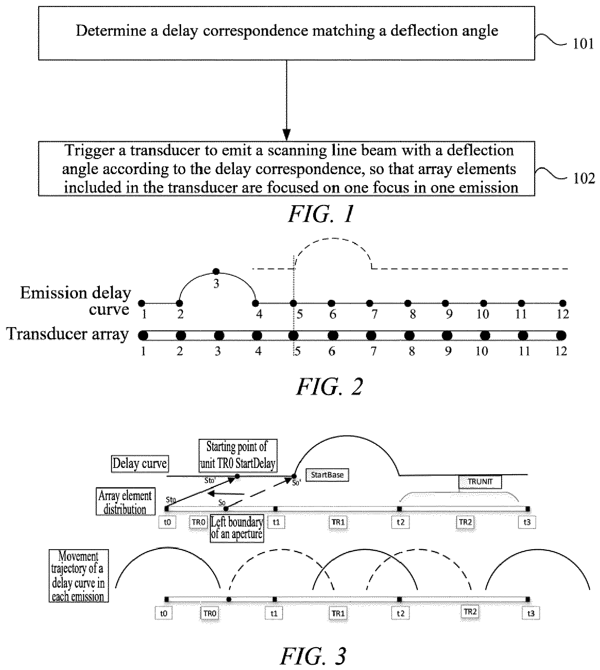

FIG. 1 shows a control method for a medical probe according to an exemplary embodiment of the present disclosure, the control method is applied to an ultrasound system, and the ultrasound system includes a transducer.

Refer to FIG. 1. The control method includes the following steps:

In step 101, a delay correspondence matching a deflection angle is determined.

In this embodiment, the delay correspondence corresponding to each deflection angle is prestored, and each deflection angle corresponds to one delay correspondence. When an emission is performed, a corresponding delay correspondence is matched based on a deflection angle of this emission.

The delay correspondence represents a correspondence between an array element number included in the transducer and an emission delay time. The delay correspondence may be implemented in a form of a table or in a form of a curve. This is not particularly limited in the present disclosure.

In step 102, the transducer is triggered to emit a scanning line beam with a deflection angle according to the delay correspondence, so that scanning line beams emitted by array elements included in the transducer are focused on one focus in one emission.

Scanning line beams with the same deflection angle are emitted based on the same delay correspondence. That is, for the same deflection angle, a delay correspondence is shared, and an emission delay time of an array element for each emission is determined based on the delay correspondence for each emission, which requires a small amount of calculation.

It may be understood that, in one emission, not all array elements participate in emitting scanning line beams, but some array elements are selected in sequence to emit scanning line beams with a deflection angle, and a plurality of emissions are performed until an ultrasound examination is completed. Although when single-focus focusing imaging is used, each time a focus position is changed, a scanning line beam needs to be emitted at a new deflection angle, scanning line beams are emitted at the same deflection angle for some emissions, and because a deflection angle, a delay time, and a focus position of each emission by the transducer are correlated, for the same deflection angle, the delay correspondence can be shared. After emitting a scanning line beam, the transducer further collects scanned image data, and the scanned image data is used to reconstruct a medical image.

In the prior art, each emission corresponds to one delay correspondence. If there are M deflection angles, N emissions may be performed at M deflection angles. M*N delay correspondences need to be stored.

In this embodiment, for a same deflection angle, the transducer shares one delay correspondence to emit a scanning line beam for each emission. It is assumed that N emissions are performed at M deflection angles for one ultrasound examination. Then, only M delay correspondences need to be stored.

In an actual application process, the scanning line beam is emitted at a deflection angle. In addition to emitting a scanning line beam to the left and right (emission with a deflection angle), a scanning line beam is actually emitted once in the middle (emission without a deflection angle), and correspondingly, a delay correspondence is added. Even so, only M+1 delay correspondences need to be stored, which is much less than M*N.

In this embodiment, a delay correspondence is shared for a same deflection angle, which can reduce a number of delay correspondences stored and save storage space, and for different focus positions, there is no need to re-calculate an emission delay time of each array element based on the focus positions, which can improve the emission efficiency.

In one embodiment, the transducer includes a transducer array, and the transducer array includes a scanning unit. The step of the transducer being triggered to emit a scanning line beam with a deflection angle according to the delay correspondence includes: determining, based on a boundary position of an effective emission aperture of one emission by the scanning unit and the delay correspondence, an emission delay time of an array element included in the scanning unit, and triggering the array element to emit the scanning line beam with the emission delay time.

A boundary position of an effective emission aperture of each emission is preset. The boundary position includes a starting position of the effective emission aperture and/or an end position of the effective emission aperture. The starting position is represented by a starting array element position of the effective emission aperture of one emission by the scanning unit. The end position is represented by an end array element position of the effective emission aperture of one emission by the scanning unit.

During single-focus focusing imaging, in one emission, a scanning line beam is generally emitted by some array elements of the transducer array. Therefore, the transducer array is divided into at least one scanning unit, and all of the scanning unit is triggered in sequence to send the scanning line beam.

One emission corresponds to one Transmit Receive Time (TRTime). Within one TRTime, one scanning unit emits a scanning line beam once and performs echo (scanned image data) collection once.

It should be noted that for one emission, scanning line beams may be emitted by all array elements included in the scanning unit, or scanning line beams may be emitted by some array elements included in the scanning unit. Similarly, for one echo collection, the collection may be performed by all the array elements included in the scanning unit, or by some of the array elements included in the scanning unit. This is not particularly limited in this embodiment of the present disclosure.

In this embodiment, one deflection angle corresponds to one delay correspondence. Through the boundary position of the effective emission aperture of the scanning unit and the matching delay correspondence, the emission delay time of the array element of the scanning unit may be determined for each emission, to share delay correspondences.

The following describes an implementation for determining the emission delay time of the array element when the delay correspondence is shared: An index between each array element included in the scanning unit and each array element number represented by the delay correspondence is determined based on the boundary position of the effective emission aperture, and the emission delay time of the array element is matched from the delay correspondence based on the index.

Specifically, the step of determining the index includes: determining the starting array element position of the effective emission aperture and a first-array element position of the scanning unit, and determining, based on the starting array element position, the first-array element position, and a starting array element number for an effective delay in the delay correspondence, an index between each array element included in the scanning unit and each array element number represented by the delay correspondence.

In order to facilitate calculation, a position of each array element included in the scanning unit is represented by a number, and the index between each array element included in the scanning unit and each array element number represented by the delay correspondence is determined, that is, an index between a number of each array element included in the scanning unit and each array element number represented by the delay correspondence is determined.

For ease of understanding, an implementation process of determining an index between each array element of the transducer array and each array element number represented by the delay correspondence will be further described by using an example in which the delay correspondence is represented by a curve.

Referring to FIG. 2, the abscissa of an emission delay curve represents an array element number, and the ordinate represents an emission delay time corresponding to each array element number. The array element number represented by the emission delay curve corresponds to the number of each array element of the transducer array. An arc part of the emission delay curve represents an effective delay of the emission delay curve. The effective delay corresponds to the effective emission aperture of one emission by the scanning unit. The effective delay represents emission delay times of all array elements within a range of the effective emission aperture of one emission.

In one embodiment, refer to FIG. 2 and FIG. 3. A number of a starting array element of the effective emission aperture of one emission by the scanning unit is 2 ($S_0$), an arc part of the emission delay curve corresponds to array elements numbered 2 to 4, that is, array element 2, array element 3, and array element 4, which are the effective emission aperture of this emission by the scanning unit. Boundary positions of the effective emission aperture of the scanning unit are respectively positions of array element 2 and array element 4. For example, the emission delay time is determined by using the starting position of the effective emission aperture. A position of array element 2 ($S_0$) is a starting array element position of the effective emission aperture of this emission, and a starting array element of an effective delay on the emission delay curve is array element 2 ($S'_0$). Then, an emission delay time of array element 2 ($S'_0$) on the emission delay curve is used as an emission delay time of array element 2 ($S_0$) of the scanning unit. Since the emission delay curve corresponds to numbers of array elements of the transducer array, according to arrangement rules of the array elements of the transducer array, emission delay times of all array elements within the range of the effective emission aperture of one emission can be found on the emission delay curve. The array elements are triggered to perform one emission based on the emission delay times, so that scanning line beams emitted by the array elements are focused on one focus at the same time in one emission.

In one embodiment, a number of a starting array element of the effective emission aperture of one emission by the scanning unit is 5 ($S_0$), a pitch delt between array element 5 ($S_0$) and array element 1 ($t_0$) is 5-1, and array element 1 ($t_0$) is a starting point array element of the transducer array. In order to share the emission delay curve represented by the solid line in FIG. 2, array element 5 ($S_0$) of the transducer array is made to correspond to array element 2 ( $S_0'$ ) on the emission delay curve, and array element 4 of the transducer array is made to correspond to a number ( $S_{t0}'$ ) of the first array element on the emission delay curve, which is equivalent to moving the emission delay curve to the right by three pitches (the dashed line in the figure represents the emission delay curve after moving to the right). Based on the arrangement rules of the array elements, emission delay times of all array elements within the range of the effective emission aperture of a next emission can be found on the emission delay curve.

A difference between the starting point array element and the starting array element is as follows: The starting point array element is the first array element of the transducer array, and has a fixed position; the starting array element is the first array element of the effective emission aperture of one emission, and has a position changing dynamically.

Based on the above example, an index between a number of each array element in each emission and each array element number represented by the emission delay curve (delay correspondence) can be deduced. Based on the index, emission delay times of all array elements within the effective emission aperture of each emission may be calculated. The array element number on the emission delay curve and the number of each array element of the transducer array may be defined artificially, or may be represented by coordinates of the array element in a coordinate system.

Through the above analysis, the following formula can be used to represent an index between an array element of the transducer array and an array element number represented by the delay correspondence:

$$S_{t0}' = S_{0'} - (S_0 - t_0).$$

Herein, $t_0$ represents a number of the starting point array element of the transducer array. $S_0$ represents a number of the starting array element of the effective emission aperture of the scanning unit of one emission. $S_{0'}$ represents the starting array element number of the effective delay on the emission delay curve. The starting array element number of the effective delay on an emission delay curve is a fixed value. $S_{t0}'$ represents an array element number corresponding to the starting point array element of the scanning unit in one emission on the delay curve.

It should be noted that for each emission, $S_{0'}$ is a fixed value, there is a case in which a calculation result of $S_{t0}'$ is less than 0, and when an emission delay time is matched on the emission delay curve, the number on the emission delay curve is cycled. For example, as shown in FIG. 2, $S_{0'}$ is 2, $S_0$ is 5, and $t_0$ is 1. $S_{t0}' = -2$ is obtained. Matching needs to be performed from the right end of the emission delay curve, that is, when $S_{t0}'$ is less than 0, the following calculation needs to be performed: -2+12=10. When the emission delay time is matched on the emission delay curve, array element number 10 on the emission delay curve is used as a number corresponding to the starting point array element of the scanning unit in this emission on the delay curve: That is, array element number 10 on the emission delay curve is made to correspond to array element 1 of the transducer array; array element number 11 on the emission delay curve is made to correspond to array element 2 of the transducer array; array element number 12 on the emission delay curve is made to correspond to array element 3 of the transducer array; array element number 1 on the emission delay curve is made to correspond to array element 4 of the transducer array; array element number 2 on the emission delay curve is made to correspond to array element 5 of the transducer array, and an emission delay time of array element number 2 on the emission delay curve is used as an emission delay time of array element 5 of the transducer array; array element number 3 on the emission delay curve is made to correspond to array element 6 of the transducer array, and an emission delay time of array element number 3 on the emission delay curve is used as an emission delay time of array element 6 of the transducer array; and so on.

The above description is made by using an example that the index is determined by using the starting array element position of the effective emission aperture of the scanning unit. In practical applications, the index may be alternatively determined based on an end array element position of the scanning unit. A specific implementation process is similar and will not be described here again.

In one embodiment, the ultrasound system further includes an emission channel, the transducer array is connected to the emission channel, and the transducer array is triggered to emit a scanning line beam through the emission channel.

In one scenario, the performance of the controller (such as an FPGA) configured to control the medical probe is limited. A number of emission channels that the controller controls at the same time is limited. The number of emission channels that the controller can control at the same time is less than a number of emission channels corresponding to one scanning unit. That is, a number of array elements included in the effective delay part (the arc part in the figure) of the emission delay curve is less than a number of array elements included in one scanning unit.

In another scenario, a number of emission channels that the controller can control at the same time is more than a number of emission channels corresponding to one scanning unit and less than a number of emission channels corresponding to two scanning units. That is, a number of array elements included in the effective delay part (the arc part in the figure) of the emission delay curve is more than a number of array elements included in one scanning unit and less than a number of all array elements included in two scanning units.

In another scenario, a number of emission channels that the controller can control at the same time is more than a number of emission channels corresponding to two scanning units. That is, a number of array elements included in the effective delay part (the arc part in the figure) of the emission delay curve is more than a number of all array elements included in two scanning units.

For the above three scenarios, the starting array element position of each emission falls into different scanning units, and the scanning units are generally controlled independently, and an index needs to be determined separately for each scanning unit. The following uses an example in which the transducer array includes three scanning units for further description.

The three scanning units are TR0, TR1, and TR2 respectively. When the number of array elements included in the effective delay part of the emission delay curve (the arc part in the figure) is less than the number of array elements included in one scanning unit, then the starting array element position of the effective emission aperture in at least one emission falls into array elements included in scanning unit TR0, an index between an array element of scanning unit TR0 and the array element number represented by the delay correspondence may be first determined, and then indexes between array elements of scanning unit TR1 and scanning unit TR2 and array element numbers represented by the delay correspondence may be deduced based on a number of emission channels in one emission; when the number of array elements included in the effective delay part of the emission delay curve is more than the number of array elements included in one scanning unit and less than the number of array elements included in two scanning units, then the starting array element position of the effective emission aperture in at least one emission falls into array elements included in scanning unit TR1, an index between an array element of scanning unit TR1 and the array element number represented by the delay correspondence may be first determined, and then indexes between array elements of scanning unit TR0 and scanning unit TR2 and array element numbers represented by the delay correspondence may be deduced based on a number of emission channels in one emission; when the number of array elements included in the effective delay of the emission delay curve is more than the number of array elements included in two scanning units, then the starting array element position of the effective emission aperture in at least one emission falls into array elements included in scanning unit TR2, an index between an array element of scanning unit TR2 and the array element number represented by the delay correspondence may be first determined, and then indexes between array elements of scanning unit TR0 and scanning unit TR1 and array element numbers represented by the delay correspondence may be deduced based on a number of emission channels in one emission.

For different scenarios, with reference to FIG. 2 and FIG. 3, the following formula can be used to represent an index between an array element of the scanning unit and the array element number represented by the delay correspondence in different scenarios.

When the starting array element position of the effective emission aperture in one emission falls within scanning unit TR0, the index between the array element number of the transducer array and the array element number represented by the delay correspondence is:

$$\begin{cases} S'_{t0} = S_{0'} - (S_0 - t_0) \\ S'_{t1} = S'_{t0} + TRUNIT \\ S'_{t2} = S'_{t0} + 2 * TRUNIT \end{cases}.$$

When the starting array element position of the effective emission aperture in one emission falls within scanning unit TR1, the index between the array element number of the transducer array and the array element number represented by the delay correspondence is:

$$\begin{cases} S'_{t0} = S'_{t1} - TRUINT \\ S'_{t1} = S_{0'} - (S_0 - t_1) \\ S'_{t2} = S'_{t1} + TRUNIT \end{cases}.$$

When the starting array element position of the effective emission aperture falls within scanning unit TR2, the index between the array element number of the transducer array and the array element number represented by the delay correspondence is:

$$\begin{cases} S'_{t0} = S'_{t2} - 2 * TRUNIT \\ S'_{t1} = S'_{t2} - TRUNIT \\ S'_{t2} = S_{0'} - (S_0 - t_2) \end{cases}.$$

$S'_{t0}$ is a number corresponding to the starting point array element of the scanning unit TR0 in one emission on the delay curve; $S'_{t1}$ is a number corresponding to the starting point array element of the scanning unit TR1 in one emission on the delay curve; $S'_{t2}$ is a number corresponding to the starting point array element of the scanning unit TR2 in one emission on the delay curve; $t_0$ is the starting point array element position (which may be represented by an array element number) of scanning unit TR0; $t_1$ is the starting point array element position (which may be represented by an array element number) of scanning unit TR1, and $t_2$ is the starting point position (which may be represented by an array element number) of scanning unit TR2; $S_0$ is a number of the starting array element of the effective emission aperture of the scanning unit of one emission; TRUNIT is a number of emission channels in one emission. $S_{t0}'$, $S_{t1}'$, and $S_{t2}'$ fall within a range [0, ChannelsNum], ChannelsNum refers to a total number of emission channels of the ultrasound system, and TRUNIT≤ChannelsNum.

In one embodiment, the transducer includes at least two transducer arrays. The step of the transducer being triggered to emit a scanning line beam with a deflection angle according to the delay correspondence includes: triggering each transducer array to emit a scanning line beam with a deflection angle in sequence according to the delay correspondence, so that imaging areas of two adjacently arranged transducer arrays having a same imaging section type have overlapping areas, where the imaging areas include at least one focus.

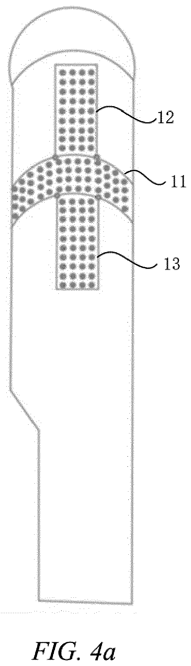

A number of transducer arrays included in the transducer, an arrangement manner of each transducer array, and an array division manner may be set according to the actual situation, and are not particularly limited in this embodiment of the present disclosure. When the transducer includes two transducer arrays, the two transducers may be arranged in an "L" shape or a "T" shape. When the transducer includes three transducer arrays, taking FIG. 4a as an example, the three transducers may be arranged in a "cross" shape. Each transducer array may be alternatively arranged in an irregular shape.

It should be noted that the array elements of the transducer may be divided into arrays in a fixed manner or dynamically according to actual needs in a scanning process.

Imaging section types of transducer arrays included in the medical probe may be the same or different. When the imaging section types of the transducer arrays included in the medical probe are all the same, for every two transducer arrays that have the same imaging section type and that are arranged adjacently to each other, each transducer array has an overlapping area with an imaging area of at least one transducer array. When the imaging section types of the transducer arrays included in the medical probe are not completely the same, it should be ensured that there is an overlapping area between imaging areas of two adjacently arranged transducer arrays that have the same imaging section type. When there is only one transducer array of one imaging section type, there is no requirement on an imaging area.

Imaging section types include a coronal plane and a sagittal plane. When the imaging section types of the transducer arrays are different, the medical probe may obtain scanned image data of a plurality of imaging sections, to display a plurality of imaging sections of a tissue in a same position in real time. For example, the medical probe may display a coronal plane and a sagittal plane of a tissue in the same position in real time, thereby reducing patient discomfort and improving clinical efficiency of clinicians.

Refer to FIG. 4a. It is assumed that imaging section types of three transducer arrays included in the medical probe are not completely the same, among which a transducer array 11 is configured to obtain scanned image data of the coronal plane, and a transducer array 12 and a transducer array 13 are both configured to obtain scanned image data of the sagittal plane. The transducer array 12 and the transducer array 13 are two target transducer arrays having the same imaging section type and are arranged adjacently to each other.

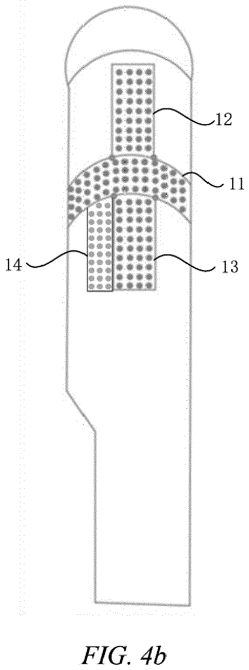

Further refer to FIG. 4b. It is assumed that imaging section types of four transducer arrays included in the medical probe are not completely the same, among which a transducer array 11 is configured to obtain scanned image data of the coronal plane, and a transducer array 12, a transducer array 13, and a transducer array 14 are all configured to obtain scanned image data of the sagittal plane. The transducer array 12 and the transducer array 13 are two target transducer arrays having the same imaging section type and are arranged adjacently to each other. The transducer array 13 and the transducer array 14 are two target transducer arrays having the same imaging section type and are arranged adjacently to each other.

Whether two transducer arrays are arranged adjacently may be defined artificially. The so-called "arranged adjacently" refers to target transducer arrays having the same imaging section type. The "arranged adjacently" does not mean that there is no transducer array having another imaging section type between two transducer arrays having the same imaging section type. For example, although the transducer array 11 is arranged between the transducer array 12 and the transducer array 13 in FIG. 4b, an imaging section type of the transducer array 11 is different from those of the transducer array 12 and the transducer array 13, and imaging section types of the transducer array 12, the transducer array 13, and the transducer array 14 are the same, the transducer array 12 and the transducer array 13 may still be defined as two target transducer arrays that have the same imaging section type and that are arranged adjacently to each other, or the transducer array 12 and the transducer array 14 may be defined as two target transducer arrays that have the same imaging section type and that are arranged adjacently to each other.

Each transducer array can obtain only part of the scanned image data of the tissue. Scanned image data obtained by target transducer arrays having a same imaging section type needs to be spliced to form a complete medical image. The prerequisite for splicing is there being an overlapping area between imaging areas of two adjacently arranged target transducer arrays that have a same imaging section type, and in this way, image splicing makes sense. Therefore, during triggering of transducer arrays to emit scanning line beams, it is required to ensure that there is an overlapping area between imaging areas of two adjacently arranged target transducer arrays that have a same imaging section type. FIG. 4b is used as an example. There is an overlapping area between imaging areas of the transducer array 12 and the transducer array 13, there is an overlapping area between imaging areas of the transducer array 13 and the transducer array 14, and there may be alternatively an overlapping area between imaging areas of the transducer array 12 and the transducer array 14.

In this embodiment, scanning line beams are emitted at a deflection angle such that there is an overlapping area between imaging areas of two adjacently arranged target transducer arrays having a same imaging section type, which is suitable for transducer arrays arranged in various arrangement manners and has good universality.

In one embodiment, two adjacently arranged target transducer arrays having the same imaging section type are triggered to emit scanning line beams by sharing array elements, so that there is an overlapping area between imaging areas of the two target transducer arrays.

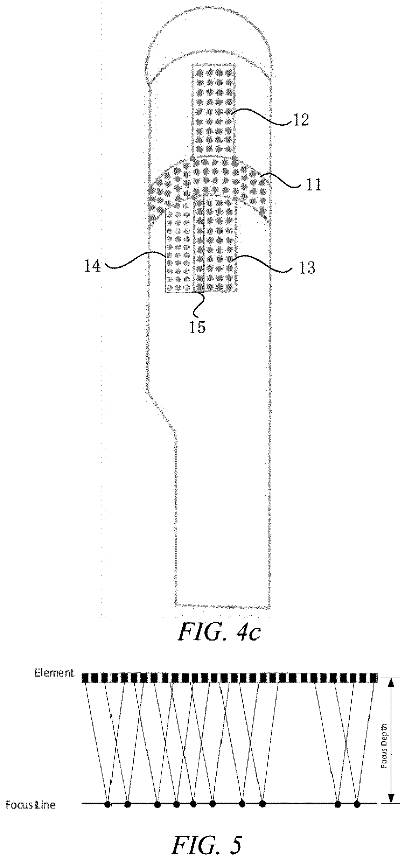

FIG. 4c is used as an example. Array elements included in a transducer array 15 in the figure are shared by the transducer array 13 and the transducer array 14, that is, the array elements included in the transducer array 15 may belong to both the transducer array 13 and the transducer array 14. When the transducer array 13 emits a scanning line beam, the transducer array 15 also emits a scanning line beam, and similarly, when the transducer array 14 emits a scanning line beam, the transducer array 15 also emits a scanning line beam, so that there is an overlapping area between imaging areas of the transducer array 13 and the transducer array 14.

It should be noted that a number of shared array elements included in the transducer array may be set according to the actual situation, provided that scanned image data obtained by transducer arrays having a same imaging section type can be effectively spliced.

In this embodiment, array elements are shared such that there is an overlapping area between imaging areas of two adjacently arranged target transducer arrays having a same imaging section type. This ensures that scanned image data obtained by transducer arrays having a same imaging section type can be effectively spliced, uses a simple control algorithm, and requires a small amount of calculation.

In one embodiment, the transducer array includes a linear transducer array. A position of focus of emission by the linear transducer array is determined based on a focus depth and a deflection angle. A scanning line beam emitted by the linear transducer array each time is focused on one focus. As shown in FIG. 5, after a plurality of emissions are performed, a focus line is formed, and each point on the focus line represents one focus.

In one embodiment, the linear transducer array includes at least one scanning unit. All of the scanning unit is triggered in sequence to emit the scanning line beam at the deflection angle. Scanning line beams emitted by array elements included in the scanning unit are focused on one focus, a focus position is determined based on a focus depth and the deflection angle, and an emission delay time represented by a delay correspondence is determined based on the focus position and a position of an array element included in the transducer.

Specifically, a delay correspondence shared for the linear transducer array is pre-established in the following manner of: determining a focus position based on a focus depth of the linear transducer array and the deflection angle, determining an emission delay time of each array element based on the focus position and a position of an array element included in the linear transducer array, and establishing the delay correspondence.

It should be noted that one emission being focused on one focus may be all array elements included in the scanning unit emitting scanning line beams that are focused on one focus, or some array elements included in the scanning unit emitting scanning line beams that are focused on one focus. A number of array elements that emit scanning line beams in one emission is determined by an effective emission aperture of one emission. The effective emission aperture may be defined according to actual needs.

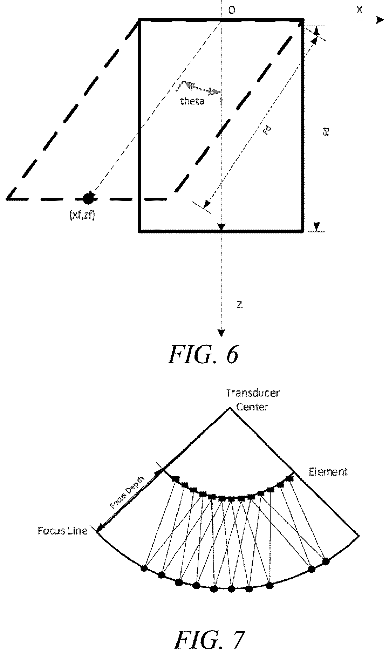

Refer to FIG. 5 and FIG. 6. For single-focus focusing imaging of a linear transducer array, a focus position of emission with a deflection angle by the linear transducer array (Element) is determined based on a focus depth and a deflection angle. Specifically, refer to FIG. 6. It is assumed that, for example, the focus and the transducer are in a same coordinate system, the array elements included in the transducer are arranged on an X-axis, and a position of a central array element of the transducer is taken as an origin O of the coordinate system. Then, a focus of emission with a deflection angle by a linear transducer array is calculated as follows:

$$\begin{cases} x_f = Ray_x + F_d \sin(\theta) \\ \phantom{xxx} z_f = F_d \cos(\theta) \end{cases}.$$

Herein, $x_f$ is the abscissa of the focus; $z_f$ is the ordinate of the focus; $Ray_x$ is the abscissa of the scanning line beam, and is represented by the abscissa of the central array element of the effective emission aperture of one emission; $F_d$ is a focus depth (a distance from the central array element of the effective emission aperture to the focus), and $\theta$ is a deflection angle (which is theta in FIG. 6). The central array element of the effective emission aperture corresponds to a central array element of the scanning unit.

When the linear transducer array performs emission with a deflection angle, in order to ensure that a scanning line beam of one emission is focused on one focus at the same time, reduce noise in scanned image data, and improve the quality of image reconstruction, each array element in one emission needs to perform emission with a specific emission delay time.

Refer to FIG. 6. When the linear transducer array performs scanning with a deflection angle, all scanning line beams are deflected with a Z-axis of the coordinate system as a reference. The deflected scanning line beams are still parallel lines, and a scanning area is a parallelogram. Under the premise that coordinates of an array element are known, for example, coordinates of an array element are (0,0), and coordinates $(x_i, z_i)$ of each remaining array element may be calculated based on a pitch between two adjacent array elements and arrangement rules of the array elements in the coordinate system. Taking a sequential number of each array element being used to represent the arrangement rules as an example, a calculation formula of each array element is as follows:

$$x_i = \text{Interval between array elements} * \text{Number of an } i^{th} \text{ array element, and } z_i = 0.$$

For example, an array element located at the origin is numbered 0, and two array elements adjacent to the array element located at the origin are numbered -1 and 1 respectively. A pitch between the two adjacent array elements is 0.6 mm. Coordinates of the two array elements are (-0.6,0) and (0.6,0) respectively. In addition to the array element at the origin, an array element adjacent to the array element numbered 1 also includes an array element numbered 2. Coordinates of this array element are (1.2,0).

In another implementation, a number of an array element is directly used as coordinates of the array element.

A pitch between array elements is determined based on sizes of the array elements in an X-axis direction and a gap between the two adjacent array elements.

A distance between the focus and each array element may be calculated using the Euler's formula d = $\sqrt{(x_f - x_i)^2 + (z_f - z_i)^2}$. Based on this distance, the emission delay time of each array element may be obtained, and a delay correspondence may be established. The emission delay time is negatively correlated with the distance.

In one embodiment, the transducer array includes a convex transducer array, and a focus position of emission by the convex transducer array is determined based on a radius and a focus depth of the convex transducer array and the deflection angle. A scanning line beam emitted by the convex transducer array each time is focused on one focus. As shown in FIG. 7, after a plurality of emissions are performed, a focus line is formed, and each point on the focus line represents one focus.

In one embodiment, the convex transducer array includes at least one scanning unit. All of the scanning unit is triggered in sequence to emit a scanning line beam at the deflection angle. Scanning line beams emitted by array elements included in the scanning unit are focused on one focus, the focus position is determined based on the radius and the focus depth of the convex transducer array and the deflection angle, and an emission delay time represented by a delay correspondence is determined based on the focus position and a position of an array element included in the transducer.

Specifically, a delay correspondence shared for the convex transducer array is pre-established in the following manner of: determining a focus position based on a radius and a focus depth of the convex transducer array and the deflection angle, determining an emission delay time of each array element based on the focus position and a position of an array element included in the convex transducer array, and establishing the delay correspondence.

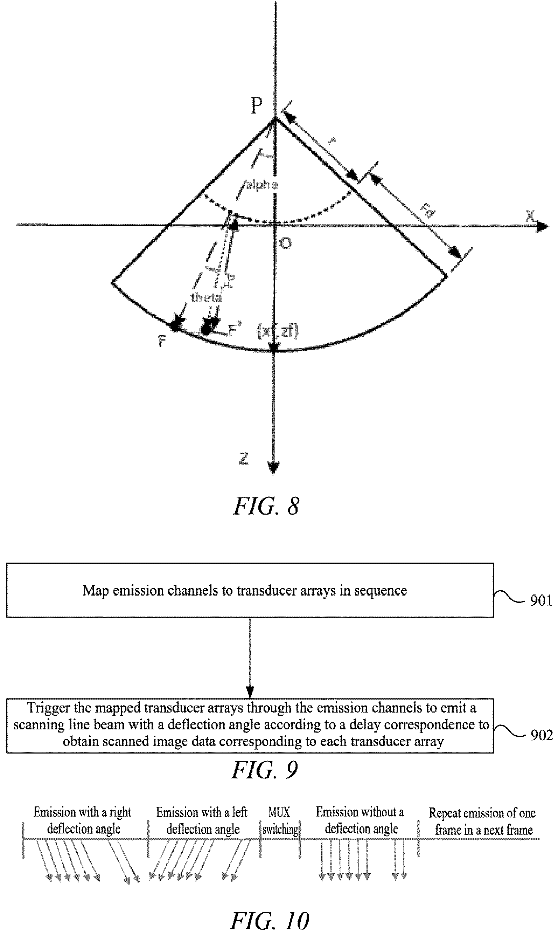

Refer to FIG. 7 and FIG. 8. For single-focus focusing imaging of a convex transducer array, a focus position of emission with a deflection angle by the convex transducer array is determined based on a radius and a focus depth of the convex transducer array and a deflection angle. Specifically, refer to FIG. 8. It is assumed that, for example, the focus and the transducer are in a same coordinate system, the array elements included in the transducer are arranged in an arc in the coordinate system, a position of a central array element of the transducer is taken as an origin O of the coordinate system, and an arc tangent with the central array element as a tangent point is taken as an X-axis. Then, a focus of emission with a deflection angle by a convex transducer array is calculated as follows:

$$\begin{cases} x_f = r * \sin(\alpha) + F_d \sin(\alpha + \theta) \\ z_f = r * \cos(\alpha) + F_d \cos(\alpha + \theta) - r \end{cases}$$

Herein, $x_f$ is the abscissa of the focus, $z_f$ is the ordinate of the focus, r is a radius of the convex transducer array, $\alpha$ (which is alpha in FIG. 8) is an angle in emission without a deflection angle, $F_d$ is a focus depth, and $\theta$ is the deflection angle (which is theta in FIG. 8).

When the convex transducer array performs emission with a deflection angle, in order to ensure that scanning line beams emitted by array elements are focused on one focus at the same time, reduce noise in scanned image data, and improve the quality of image reconstruction, each array element needs to perform emission with a specific emission delay time.

Refer to FIG. 8, when the convex transducer array performs emission with a deflection angle, an emission center of the scanning unit is a rotation center, each scanning line beam is deflected with an original normal direction (the direction in which the probe performs emission without a deflection angle) as a reference, and a scanning area formed is a sector asymmetrical on the left and right sides. A central angle of an arc segment with each array element and a central array element as endpoints may be calculated by using a formula of an arc length and a central angle L = r * β, (L is the arc length, r is a radius, and β is an angle corresponding to the arc length), and then coordinates of the central angle may be calculated.

A calculation formula of a central angle of each array element is as follows:

$$\beta_i = L_i / r.$$

Herein, $L_i$ represents an arc length of an arc segment with an $i^{th}$ array element and the central array element as endpoints; $\beta_i$ represents a central angle (that is, an angle $\alpha$ of each array element in FIG. 8) of the arc segment with the $i^{th}$ array element and the central array element as endpoints. The Transducer Center in the figure represents the center of the arc segment.

If the arc lengths between two adjacent array elements in the convex transducer array are equal, $L_i$ may be calculated according to arrangement rules of the array elements in the coordinate system.

Coordinates of each array element are:

$$\begin{cases} x_i = r * \sin(\beta_i) \\ z_i = r * \cos(\beta_i) \end{cases}$$

A distance between a focus and each array element is further calculated using the Euler's formula. Based on this distance, the emission delay time of each array element may be obtained, and a delay correspondence may be established.

In one embodiment, the transducer being triggered to emit a scanning line beam with a deflection angle according to the delay correspondence includes: mapping the emission channels to transducer arrays in sequence, and triggering the mapped transducer arrays through the emission channels to emit a scanning line beam with a deflection angle according to the delay correspondence.

In this embodiment, an emission channel and a transducer array are not connected in a fixed manner, but are in a channel multiplexing manner. During an ultrasound examination, different transducer arrays can be dynamically mapped to emission channels, and array elements of the transducer arrays mapped to the emission channels are triggered to emit scanning line beams.

The so-called "mapping" means establishing a connection between an emission channel and a transducer array. In one embodiment, a switch array is provided between the transducer array and the emission channel. The connection between the emission channel and the transducer array is established by switching states of switches in the switch array. The switch array may be implemented by, but is not limited to, a multiplexer switch MUX.

In the implementation of the present disclosure, the medical probe is controlled by channel multiplexing. Emission channels and array elements included in the transducer do not need to be in a one-to-one correspondence. Even if a number of emission channels is less than a total number of array elements included in the transducer, the medical probe can be effectively controlled, and effective scanned image data for reconstructing medical images can be collected, thereby simplifying the structure of the medical probe without affecting the quality of image reconstruction.

FIG. 9 is a flowchart of another control method for a medical probe according to an exemplary embodiment of the present disclosure. The control method includes the following steps:

In step 901, emission channels are mapped to transducer arrays in sequence.

Because the channel multiplexing manner is used, each time the emission channels are mapped to part of the transducer arrays or some array elements of one transducer array, the emission channels are mapped to the transducer arrays in sequence, that is, a connection relationship between the emission channels and the array elements included in the transducer array is established to trigger the array elements for which the connection relationship is established to the emission channels to emit scanning line beams. The above part of the transducer arrays may be one or more transducer arrays.

In step 902, the mapped transducer arrays are triggered through the emission channels to emit a scanning line beam with a deflection angle according to a delay correspondence to obtain scanned image data corresponding to each transducer array.

Scanning line beams with the same deflection angle are emitted based on the same delay correspondence. There is an overlapping area between imaging areas of two adjacently arranged target transducer arrays having a same imaging section type; the scanned image data is used to reconstruct a medical image.

FIG. 4b is used as an example. The transducer array 12 and the transducer array 13 are triggered to deflect toward each other at a preset deflection angle to emit a scanning line beam, so that there is an overlapping area between the imaging areas of the transducer array 12 and the transducer array 13.

In one implementation, a number of emission channels is less than a number of all array elements included in one transducer array. In this case, in one emission, all the array elements included in one transducer array can not be mapped to the emission channels, and instead, all the array elements included in the transducer array need to be divided into at least two scanning units based on the number of emission channels. A number of array elements included in each scanning unit is less than or equal to the number of emission channels. The scanning units are mapped to the emission channels in sequence, and each scanning unit is triggered to emit a scanning line beam.

In one implementation, the computing performance of the controller (such as an FPGA) configured to control the medical probe is limited. A number of emission channels that the controller can control at the same time is limited. When a maximum number of emission channels that the controller can control at the same time is less than the total number of emission channels, all the array elements included in the transducer array are divided into at least two scanning units based on the maximum number of emission channels that the controller can control at the same time. A number of array elements included in each scanning unit is less than or equal to the maximum number of emission channels that the controller can control at the same time. The scanning units are mapped to the emission channels in sequence, and each scanning unit is triggered to emit a scanning line beam. For example, the ultrasound system includes 192 emission channels and the maximum number of emission channels that the controller can control at the same time is 64. The transducer array is divided into a plurality of scanning units, and a number of array elements included in each scanning unit is less than or equal to the maximum number of emission channels that the controller can control at the same time. For example, the scanning unit includes 64 array elements, and 64 emission channels are mapped to 64 array elements in each emission.

In one embodiment, the control method for a medical probe in any of the above embodiments is applicable to a biplane probe.

In the following, for example, the medical probe includes a biplane probe. The biplane probe includes two first transducer arrays and one second transducer array. The control process of the medical probe is further described. The two first transducer arrays are used for sagittal plane imaging and may include two small curved convex arrays or two linear arrays, for example, may include 96 array elements, may have a frequency within 5 MHz to 12 MHz (megahertz), and may have a pitch (which is a pitch between two array elements) being 0.27 mm (millimeter), have an elevation (which is a thickness of an array element) being 4 mm, and have an ROC (curvature) being 60 mm or flat; the second transducer array is used for coronal plane imaging, and different from the type of the first transducer array, it may be made of an array transducer including 192 array elements with a frequency within 3 MHz to 10 MHz, and has a pitch being 0.16 mm, an elevation being 5 mm, and an ROC being 10 mm or flat.

FIG. 10 is a schematic diagram of an emission timing sequence of a transducer array exemplarily shown in the embodiment. The medical probe includes a MUX (multiplexer, data selector, also known as multiplexer switch) internally for emission channel multiplexing. During normal operation, emission channels are first mapped to the two first transducer arrays of the same type, and the two first transducer arrays of the same type are triggered to emit scanning line beams with a deflection angle according to a delay correspondence. There is an overlapping area between imaging areas of the two first transducer arrays of the same type. Image reconstruction is performed on scanned image data collected by the two first transducer arrays of the same type to obtain a frame of medical image of a sagittal plane. Then, the emission channels are mapped to the second transducer array through the MUX, the second transducer array is triggered to emit a scanning line beam with a deflection angle according to a delay correspondence, and image reconstruction is performed on scanned image data collected by the second transducer array to obtain a frame of medical image of a coronal plane.

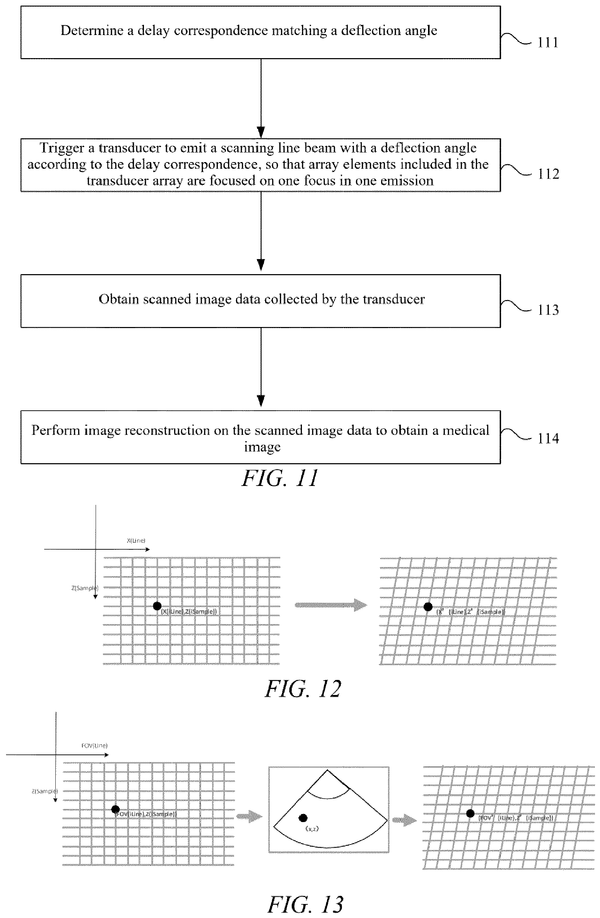

FIG. 11 is a flowchart of an imaging method according to an exemplary embodiment of the present disclosure. The imaging method includes the following steps:

In step 111, a delay correspondence matching a deflection angle is determined.

In step 112, the transducer is triggered to emit a scanning line beam with a deflection angle according to the delay correspondence, so that scanning line beams emitted by array elements included in the transducer are focused on one focus in one emission.

For a specific implementation process of step 111 and step 112, refer to the description of step 101 and step 102 in any of the above embodiments. Details are not described here again.

In step 113, scanned image data collected by the transducer is obtained.

In step 114, image reconstruction is performed on the scanned image data to obtain a medical image.

In this embodiment, during emission of the scanning line beam, a delay correspondence is shared for a same deflection angle, which can reduce a number of delay correspondences stored, and there is no need to re-calculate an emission delay time of each array element based on focus positions, which can improve the emission efficiency, and further improve the imaging efficiency.

In one embodiment, when the transducer includes at least two transducer arrays and the imaging types of the transducer arrays are not completely the same, the scanned image data collected in step 113 has different imaging section types, and during image reconstruction, image reconstruction is separately performed on scanned image data having a same imaging section type, to obtain medical images corresponding to each imaging section type.

For example, scanned image data collected in one scan includes scanned image data of a coronal plane and scanned image data of a sagittal plane, the scanned image data of the coronal plane is collected by a plurality of transducer arrays, and the scanned data of the sagittal plane is collected by one transducer array. Image reconstruction is performed on the scanned image data of the coronal plane collected by the plurality of transducer arrays to obtain a frame of medical image of the coronal plane; image reconstruction is performed on the scanned image data of the sagittal plane to obtain a frame of medical image of the sagittal plane.

It is required to perform scan transformation on the scanned image data with a deflection angle collected by the transducer, and then image splicing is performed on the scanned image data obtained after the scan transformation. Bilinear interpolation may be used for image splicing. Refer to FIG. 12. Scan transformation of the scanned image data collected by the linear transducer array is calculated as follows:

$$iLine' = \frac{x(iLine) - z(iSample) * tan\theta}{sampleSizeX};$$

$$iSample' = \frac{iSample}{cos\theta}.$$

Herein, *sampleSizeX* is an image pixel size of a sampling point of the linear transducer array in an X-axis direction; $\theta$ is the deflection angle; $x(iLine)$ represents the abscissa of an $iLine$th receiving line beam collected by the linear transducer array; $z(iSample)$ represents a depth of an $iSample$th sampling point; *iLine'* and *iSample'* represent image data obtained after scan transformation. A medical image may be presented by displaying *iLine'* and *iSample'* through a display.

During an ultrasound scan, the transducer continuously samples scanned image data. The scanned image data sampled by a specific array element over a period of time can be considered as one receiving line beam. The depth of the sampling point is positively correlated with time.

For the scanned image data with a deflection angle collected by the convex transducer array, data without a deflection angle is first transformed into a physical space and then mapped to data with a deflection angle. The first step is to transform the data without a deflection angle into the physical space:

$$\begin{cases} x = \big(r + z(iSample)\big) * \sin\big(FOV(iLine)\big) \\ z = \big(r + z(iSample)\big) * \cos\big(FOV(iLine)\big) \end{cases}.$$

Herein, $(x,z)$ represents coordinates of any point in an XZ-axis coordinate system; $r$ is a radius of a convex transducer array; $z(iSample)$ is a depth of an $iSample^{th}$ sampling point, that is, a z-coordinate of the $iSample^{th}$ sampling point on a Z-axis; $FOV(iLine)$ is an angle corresponding to an $iLine^{th}$ receiving line beam, represents an angle of the entire medical image, and is a set value.

Refer to FIG. 13. The next step is to map points in the physical space to numbers with deflection angles to obtain image data $iLine'$ and $iSample'$ after scan transformation, the calculation of which is as follows:

$$FOV' = \text{atan}\left(\frac{x}{z}\right) + \text{asin}\left(\frac{r*\sin\theta}{\sqrt{x^2+z^2}}\right) - \theta;$$

$$z' = \frac{\sqrt{x^2+z^2}}{\sin\theta} * \sin\left(\text{atan}\left(\frac{x}{z}\right) - FOV'\right);$$

$$iSample' = \frac{z'}{sampleSizeZ};$$

$$iLine' = \frac{FOV' + \frac{FOV}{2}}{sampleSizeX}.$$

Herein, $FOV'$ represents an angle with a deflection angle after mapping; $z'$ represents a z-coordinate with a deflection angle after mapping; both $FOV'$ and $z'$ are intermediate variables of scan transformation. $iLine'$ and $iSample'$ represent image data obtained after scan transformation. A medical image may be presented by displaying $iLine'$ and $iSample'$ through a display.

In this embodiment, when the above control method is used to obtain scanned image data of a scanned object, a plurality of section views of a tissue in a same position may be displayed in real time, thereby reducing patient discomfort and improving clinical efficiency of clinicians. This can reduce speckle noise and clutter in section views and enhance spatial resolution. In addition, the emission with a deflection angle during punch biopsy can increase the signal of the puncture needle, thereby completing the puncture operation accurately, reducing the costs for underlying hardware calculations, and improving the imaging frame rate. After two probes of the same type are used for emission with a deflection angle, scan transformation is performed on data with a deflection angle and then spatial fusion is performed to make up for the missing areas

Corresponding to the above embodiments of the control method for medical probe, the present disclosure further provides embodiments of an ultrasound system and an imaging system.

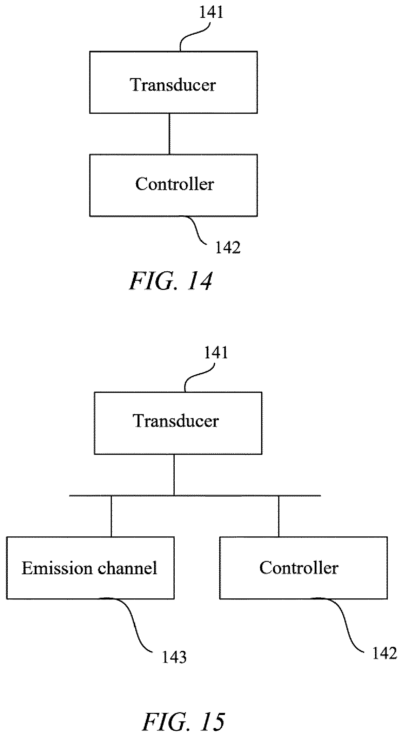

FIG. 14 is a schematic diagram of modules of an ultrasound system according to an exemplary embodiment of the present disclosure. The ultrasound system includes a transducer 141 and a controller 142. The controller 142 is configured to determine a delay correspondence matching a deflection angle, and trigger the transducer to emit a scanning line beam with a deflection angle according to the delay correspondence, so that scanning line beams emitted by array elements included in the transducer are focused on one focus in one emission, where scanning line beams with the same deflection angle are emitted based on the same delay correspondence, and the delay correspondence represents a correspondence between an array element number and an emission delay time.

In this embodiment, a delay correspondence is shared for a same deflection angle, which can reduce a number of delay correspondences stored and save storage space, and for different focus positions, there is no need to re-calculate an emission delay time of each array element based on the focus positions, which can improve the emission efficiency.

Optionally, the transducer includes a transducer array, the transducer array includes a scanning unit, and the controller 142 is configured to determine, based on a boundary position of an effective emission aperture of one emission by the scanning unit and the delay correspondence, an emission delay time of an array element included in the scanning unit, and trigger the array element to emit the scanning line beam with the emission delay time.

Optionally, the controller 142 is configured to determine, based on the boundary position of the effective emission aperture, an index between each array element included in the scanning unit and each array element number represented by the delay correspondence, and match the emission delay time of the array element from the delay correspondence based on the index.

Optionally, the transducer includes at least two transducer arrays, and the controller 142 is configured to trigger each transducer array to emit a scanning line beam with a deflection angle according to the delay correspondence, so that imaging areas of two adjacently arranged transducer arrays having a same imaging section type have overlapping areas, where the imaging areas include at least one focus.

Optionally, the transducer includes a first transducer array and a second transducer array of different types.

Optionally, there are at least two first transducer arrays, and the first transducer arrays are arranged on both sides of a length direction of the second transducer array.

Optionally, the transducer includes a linear transducer array, and a focus position of emission by the linear transducer array is determined based on a focus depth and the deflection angle; the emission delay time of the delay correspondence is determined based on the focus position and a position of an array element included in the linear transducer array.

Specifically, a delay correspondence shared for the linear transducer array is pre-established in the following manner of: determining a focus position based on a focus depth of the linear transducer array and the deflection angle, determining an emission delay time of each array element based on the focus position and a position of an array element included in the linear transducer array, and establishing the delay correspondence;

and/or the transducer includes a convex transducer array, and a focus position of emission by the convex transducer array is determined based on a radius and a focus depth of the convex transducer array and the deflection angle; the emission delay time of the delay correspondence is determined based on the focus position and a position of an array element included in the convex transducer array.

Specifically, a delay correspondence shared for the convex transducer array is pre-established in the following manner of: determining a focus position based on a radius and a focus depth of the convex transducer array and the deflection angle, determining an emission delay time of each array element based on the focus position and a position of an array element included in the convex transducer array, and establishing the delay correspondence.

FIG. 15 is a schematic diagram of modules of another ultrasound system according to an exemplary embodiment of the present disclosure. The ultrasound system includes a transducer 141, a controller 142, and an emission channel 143. The transducer 141 includes at least two transducer arrays. The controller 142 is configured to map the emission channels 143 to transducer arrays in sequence, and trigger the mapped transducer arrays through the emission channels 143 to emit a scanning line beam with a deflection angle according to the delay correspondence.

In the implementation of the present disclosure, the medical probe is controlled by channel multiplexing. Emission channels and array elements included in the transducer do not need to be in a one-to-one correspondence. Even if a number of emission channels is less than a total number of array elements included in the transducer, the medical probe can be effectively controlled, and effective scanned image data for reconstructing medical images can be collected, thereby simplifying the structure of the medical probe without affecting the quality of image reconstruction.

Optionally, the ultrasound system further includes a switch array, the transducer is connected to the emission channels through the switch array.

The controller 142 is configured to switch states of switches in the switch array to map the emission channels to the transducer array in sequence.

Optionally, the transducer includes a transducer array for coronal plane imaging and a transducer array for sagittal plane imaging.

Because system embodiments basically correspond to the method embodiments, reference may be made to the description of the method embodiments for the related parts. The system embodiments described above are merely exemplary. The units described as separate parts may or may not be physically separated, and the parts displayed as units may or may not be physical units, that is, may be located in one place, or may be distributed over a plurality of network units. Some or all of the modules can be selected according to actual needs to achieve the objectives of solutions of the present disclosure.

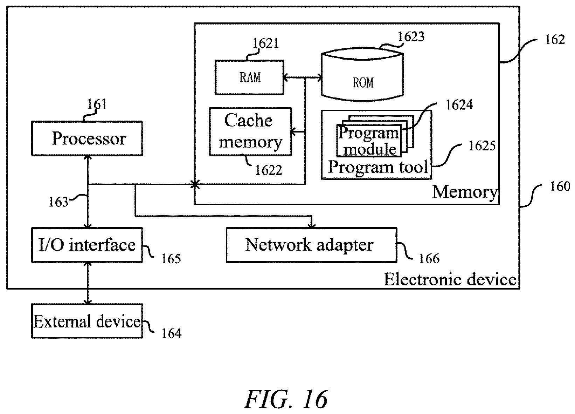

FIG. 16 is a schematic diagram of a structure of an electronic device according to an exemplary embodiment of the present disclosure, and is a block diagram of an exemplary electronic device 160 that is suitable for implementing embodiments of the present disclosure. The electronic device 160 shown in FIG. 16 is only an example and should not bring any limitations to the functions and scope of use of the embodiments of the present disclosure.

As shown in FIG. 16, the electronic device 160 may be in a form of a general computing device, which may be, for example, a server device. Components of the electronic device 160 may include, but are not limited to: at least one processor 161, at least one memory 162, and a bus 163 that connects different system components (including the memory 162 and the processor 161).

The bus 163 includes a data bus, an address bus, and a control bus.

The memory 162 may include a volatile memory, for example, a random access memory (RAM) 1621 and/or a cache memory 1622, and may further include a read-only memory (ROM) 1623.

The memory 162 may include a program tool (or utility tool) 1625 having a set of (at least one) program modules 1624, such program modules 1624 including but not limited to: an operating system, one or more applications, and other program modules and program data, where each of or a certain combination of these examples may include the implementation of a network environment.

The processor 161 runs a computer program stored in the memory 162 so as to execute various functional applications and perform data processing, for example, perform the method provided in any of the above embodiments.

The electronic device 160 may also communicate with one or more external devices 164 (for example, a keyboard, a pointing device, etc.). This communication can be performed through an input/output (I/O) interface 165. In addition, the electronic device 160 may further communicate with one or more networks (for example, a local area network (LAN), a wide area network (WAN), and/or a public network, such as the Internet) through a network adapter 166. As shown in the figure, the network adapter 166 communicates with other modules of the electronic device 160 through the bus 163. It should be understood that, although not shown in the figure, other hardware and/or software modules may be utilized in conjunction with the electronic device 160, including but not limited to: microcode, a device driver, a redundant processor, an external disk drive array, a RAID (disk array), a tape drive, a data backup storage system, and the like.

It should be noted that, even though several units/modules or sub-units/sub-modules of the electronic device are mentioned in the above detailed description, such a division is exemplary and not mandatory. Actually, according to the implementations of the present disclosure, the features and functions of two or more units/modules described above may be embodied in one unit/module. Conversely, the features and functions of one unit/module described above can be further divided into and embodied by a plurality of units/modules.

An embodiment of the present disclosure further provides a computer-readable storage medium with a computer program stored therein, and when the program is executed by a processor, the method provided in any of the above embodiments is implemented.

More specifically, the readable storage medium may include, but are not limited to: a portable disk, a hard disk, a random access memory, a read-only memory, an erasable programmable read-only memory, an optical storage device, a magnetic storage device, or any suitable combination thereof.

In a possible implementation, an embodiment of the present disclosure may be alternatively implemented in the form of a program product including program code used, when the program product is running on a terminal device, to cause the terminal device to implement the method in any of the above embodiments.

The program code for performing the present disclosure may be written in any combination of one or more programming languages. The program code may be entirely executed on user equipment, partially executed on the user equipment, executed as an independent software package, partially executed on the user equipment and partially executed on a remote device, or entirely executed on the remote device.

It is worth noting that those skilled in the art should know that the embodiments disclosed in the present application may be extended to a case of four or more scanning units. Therefore, a delay sharing method of a transducer array including a plurality of scanning units shall fall within the scope of protection of the present disclosure.

Although the specific embodiments of the present disclosure have been described above, it will be understood by those of skill in the art that these are merely illustrative, and that various alterations or modifications can be made to these embodiments without departing from the principle and essence of the present disclosure. Therefore, the scope of protection of the present disclosure is defined by the appended claims.

**Claims**

1. A control method for a medical probe, **characterized in that** the control method is applied to an ultrasound system, the ultrasound system comprises a transducer, and the control method comprises:

   determining, for one emission, a delay correspondence matching a deflection angle; and
   triggering the transducer to emit scanning line beams with the deflection angle according to the delay correspondence, so that scanning line beams emitted by array elements comprised in the transducer are focused on one focus in one emission,
   wherein scanning line beams with the same deflection angle are emitted based on the same delay correspondence, and the delay correspondence represents a correspondence between an array element number and an emission delay time.

2. The control method for the medical probe according to claim 1, **characterized in that** the transducer comprises a transducer array, the transducer array comprises a scanning unit, and the triggering the transducer to emit scanning line beams with the deflection angle according to the delay correspondence comprises:

   determining, based on a boundary position of an effective emission aperture of one emission by the scanning unit and the delay correspondence, an emission delay time of an array element comprised in the scanning unit;

triggering the array element to emit the scanning line beam with the emission delay time.

3. The control method according to claim 2, **characterized in that** the determining, based on a boundary position of an effective emission aperture of one emission by the scanning unit and the delay correspondence, an emission delay time of an array element comprised in the scanning unit comprises:

determining, based on the boundary position of the effective emission aperture, an index between each array element comprised in the scanning unit and each array element number represented by the delay correspondence;

matching the emission delay time of the array element comprised in the scanning unit from the delay correspondence based on the index.

4. The control method according to claim 3, **characterized in that** the boundary position comprises a starting array element position, and the determining, based on the boundary position of the effective emission aperture, an index between each array element comprised in the scanning unit and each array element number represented by the delay correspondence comprises:

determining the starting array element position and a first-array element position of the scanning unit;

determining, based on the starting array element position, the first-array element position, and a starting array element number for an effective delay in the delay correspondence, the index between each array element comprised in the scanning unit and each array element number represented by the delay correspondence.

5. The control method for the medical probe according to any one of claims 1 to 4, **characterized in that** the transducer comprises at least two transducer arrays, and the triggering the transducer to emit scanning line beams with the deflection angle according to the delay correspondence comprises:

triggering each of the transducer arrays to emit scanning line beams with the deflection angle according to the delay correspondence, so that imaging areas of two adjacently arranged transducer arrays having a same imaging section type have overlapping areas.

6. The control method for the medical probe according to any one of claims 1 to 5, **characterized in that** the transducer comprises a linear transducer array, and the delay correspondence is pre-established in the following manner of: determining a focus position based on a focus depth of the linear transducer array and the deflection angle, determining an emission delay time of each array element based on the focus position and a position of an array element comprised in the linear transducer array, and establishing the delay correspondence;

and/or the transducer comprises a convex transducer array, and the delay correspondence is pre-established in the following manner of: determining a focus position based on a radius and a focus depth of the convex transducer array and the deflection angle, determining an emission delay time of each array element based on the focus position and a position of an array element comprised in the convex transducer array, and establishing the delay correspondence.

7. The control method for the medical probe according to any one of claims 1 to 6, **characterized in that** the ultrasound system further comprises emission channels, the transducer comprises at least two transducer arrays, and the triggering the transducer to emit scanning line beams with the deflection angle according to the delay correspondence comprises:

mapping the emission channels to the transducer arrays in sequence, and triggering the mapped transducer arrays through the emission channels to emit scanning line beams with the deflection angle according to the delay correspondence.

8. An imaging method, **characterized in that** the imaging method comprises:

obtaining, for one emission, scanned image data collected by a transducer; the scanned image data being collected by controlling the transducer comprised in the medical probe by the control method for the medical probe according to any one of claims 1 to 7;

performing image reconstruction on the scanned image data to obtain a medical image.

9. The imaging method according to claim 8, **characterized in that** the performing image reconstruction on the scanned image data comprises:

performing image reconstruction on scanned image data having a same imaging section type, to obtain a medical image corresponding to the imaging section type.

10. An ultrasound system, **characterized in that** the ultrasound system comprises a transducer and a controller, wherein the controller is configured to determine, for one emission, a delay correspondence matching a deflection angle, and trigger the transducer to emit scanning line beams with the deflection angle according to the delay correspondence, so that scanning line beams emitted by array elements comprised in the transducer are focused on one focus in one emission,

    wherein scanning line beams with the same deflection angle are emitted based on the same delay correspondence, and the delay correspondence represents a correspondence between an array element number and an emission delay time.

11. The ultrasound system according to claim 10, **characterized in that** the transducer comprises a transducer array, the transducer array comprises a scanning unit, and the controller is configured to determine, based on a boundary position of an effective emission aperture of one emission by the scanning unit and the delay correspondence, an emission delay time of an array element comprised in the scanning unit, and trigger the array element to emit the scanning line beams with the emission delay time.

12. The ultrasound system according to claim 11, **characterized in that** the controller is configured to determine, based on the boundary position of the effective emission aperture, an index between each array element comprised in the scanning unit and each array element number represented by the delay correspondence, and match the emission delay time of the array element comprised in the scanning unit from the delay correspondence based on the index.

13. The ultrasound system according to any one of claims 10 to 12, **characterized in that** the transducer comprises at least two transducer arrays, and the controller is configured to trigger each of the transducer arrays to emit scanning line beams with the deflection angle according to the delay correspondence, so that imaging areas of two adjacently arranged transducer arrays having a same imaging section type have overlapping areas.

14. The ultrasound system according to claim 13, **characterized in that** the transducer comprises a first transducer array and a second transducer array of different types;

    there are at least two first transducer arrays, and the first transducer arrays are arranged on both sides of a length direction of the second transducer array.

15. The ultrasound system according to any one of claims 10 to 14, **characterized in that** the ultrasound system further comprises emission channels, the transducer comprises at least two transducer arrays, and the controller is configured to map the emission channels to the transducer arrays in sequence, and trigger the mapped transducer arrays through the emission channels to emit scanning line beams with the deflection angle according to the delay correspondence;

    preferably, the ultrasound system further comprises a switch array, the transducer is connected to the emission channel through the switch array, and the controller switches states of switches in the switch array to map the emission channels to the transducer arrays in sequence.

**FIG. 1**

**FIG. 2**

**FIG. 3**

FIG. 4a

**EP 4 394 442 A1**

*FIG. 4b*

23

*FIG. 4c*

Element

Focus Depth

Focus Line

*FIG. 5*

*FIG. 6*

*FIG. 7*

*FIG. 8*

| Map emission channels to transducer arrays in sequence | ⌇ 901 |
|---|---|

↓

| Trigger the mapped transducer arrays through the emission channels to emit a scanning line beam with a deflection angle according to a delay correspondence to obtain scanned image data corresponding to each transducer array | ⌇ 902 |
|---|---|

*FIG. 9*

| Emission with a right deflection angle | Emission with a left deflection angle | MUX switching | Emission without a deflection angle | Repeat emission of one frame in a next frame |
|---|---|---|---|---|

*FIG. 10*

Determine a delay correspondence matching a deflection angle  111

Trigger a transducer to emit a scanning line beam with a deflection angle according to the delay correspondence, so that array elements included in the transducer array are focused on one focus in one emission  112

Obtain scanned image data collected by the transducer  113

Perform image reconstruction on the scanned image data to obtain a medical image  114

*FIG. 11*

*FIG. 12*

*FIG. 13*

141

Transducer

Controller

142

*FIG. 14*

141

Transducer

Emission channel

Controller

143

142

*FIG. 15*

*FIG. 16*

Europäisches
Patentamt

European
Patent Office

Office européen
des brevets

# EUROPEAN SEARCH REPORT

Application Number

EP 23 22 0440

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2007/239001 A1 (MEHI JAMES [CA] ET AL) 11 October 2007 (2007-10-11) <br> * claim 24; figure 16 * <br> * paragraphs [0005], [0140], [0141] * <br> * paragraphs [0143], [0144], [0146] * <br> * paragraph [0147] – paragraph [0152] * <br> * paragraphs [0187], [0189], [0190] * <br> ----- | 1-15 | INV. <br> G01S7/52 <br> G01S15/89 <br> A61B8/00 |

**TECHNICAL FIELDS SEARCHED (IPC)**

G01S
A61B

The present search report has been drawn up for all claims

| Place of search | Date of completion of the search | Examiner |
|---|---|---|
| Munich | 24 April 2024 | Knoll, Bernhard |

EPO FORM 1503 03.82 (P04C01)

## ANNEX TO THE EUROPEAN SEARCH REPORT
## ON EUROPEAN PATENT APPLICATION NO.

EP 23 22 0440

This annex lists the patent family members relating to the patent documents cited in the above-mentioned European search report.
The members are as contained in the European Patent Office EDP file on
The European Patent Office is in no way liable for these particulars which are merely given for the purpose of information.

24-04-2024

| Patent document cited in search report | | Publication date | Patent family member(s) | | Publication date |
|---|---|---|---|---|---|
| US 2007239001 | A1 | 11-10-2007 | CA | 2628100 A1 | 18-05-2007 |
| | | | CA | 2935422 A1 | 18-05-2007 |
| | | | CN | 101351724 A | 21-01-2009 |
| | | | EP | 1952175 A2 | 06-08-2008 |
| | | | ES | 2402741 T3 | 08-05-2013 |
| | | | HK | 1129243 A1 | 20-11-2009 |
| | | | JP | 5630958 B2 | 26-11-2014 |
| | | | JP | 5690900 B2 | 25-03-2015 |
| | | | JP | 2009514600 A | 09-04-2009 |
| | | | JP | 2014000465 A | 09-01-2014 |
| | | | JP | 2014210201 A | 13-11-2014 |
| | | | JP | 2017035528 A | 16-02-2017 |
| | | | US | RE46185 E | 25-10-2016 |
| | | | US | 2007239001 A1 | 11-10-2007 |
| | | | WO | 2007056104 A2 | 18-05-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202211715857

- CN 202311707871